# EUROPEAN PATENT SPECIFICATION

(11) **EP 4 079 739 B1**
(45) Date of publication and mention of the grant of the patent: **09.12.2026**
(21) Application number: 20901094.1
(22) Date of filing: 26.10.2020
(51) Int. Cl.: A61K 31/675, A61K 31/683, A61P 31/22, A61K 45/06, C07F 9/6561, A61K 31/192

(54) **ADEFOVIR AND ADEFOVIR DIPIVOXIL FOR USE IN TREATING PSEUDORABIES VIRUS**
ADEFOVIR UND ADEFOVIRDIPIVOXIL ZUR BEHANDLUNG VON PSEUDORABIESVIRUS
ADÉFOVIR ET ADÉFOVIR DIPIVOXIL POUR UTILISATION DANS LE TRAITEMENT DU VIRUS DE LA PSEUDORAGE

(30) Priority: 20.12.2019 CN 201911326353
(43) Date of publication of application: 26.10.2022
(73) Proprietor: Xiamen University, Xiamen, Fujian 361005 (CN); Yang Sheng Tang Company, Ltd., Hangzhou, Zhejiang 310000 (CN)
(72) Inventor: CHEN, Yixin, XIAMEN, Fujian 361005 (CN); WANG, Guosong, XIAMEN, Fujian 361005 (CN); HUANG, Pengfei, XIAMEN, Fujian 361005 (CN); CHEN, Ruiqi, XIAMEN, Fujian 361005 (CN); LIN, Lina, XIAMEN, Fujian 361005 (CN); HAN, Qiangyuan, XIAMEN, Fujian 361005 (CN); XIA, Ningshao, XIAMEN, Fujian 361005 (CN)
(74) Representative: Meissner Bolte Partnerschaft mbB
(86) International application number: PCT/CN2020/123641
(87) International publication number: WO 2021/120850

(56) References cited:
- WO-A1-2007/068380
- CN-A- 101 781 333
- CN-A- 106 974 985
- CN-A- 109 758 487
- KURODA YUDAI ET AL: "Antiviral effect of sinefungin on in vitro growth of feline herpesvirus type 1", THE JOURNAL OF ANTIBIOTICS, NATURE PUBLISHING GROUP UK, LONDON, vol. 72, no. 12, 18 September 2019 (2019-09-18), pages 981 - 985, XP037923422, ISSN: 0021-8820, [retrieved on 20190918], DOI: 10.1038/S41429-019-0234-4
- ANGARANO G: "COMING THERAPIES: ADEFOVIR", INTERNATIONAL JOURNAL OF CLINICAL PRACTICE. SUPPLEMENT, MEDICOM INTERNATIONAL, ESHER, GB, vol. 103, 1 June 1999 (1999-06-01), pages 39 - 41, XP009038410, ISSN: 1368-504X
- ZOUHAROVA DARINA; LIPENSKA IVANA; FOJTIKOVA MARTINA; KULICH PAVEL; NECA JIRI; SLANY MICHAL; KOVARCIK KAMIL; TURANEK-KNOTIGOVA PAVL: "Antiviral activities of 2, 6-diaminopurine-based acyclic nucleoside phosphonates against herpesviruses: In vitro study results with pseudorabies virus (PrV, SuHV-1)", VETERINARY MICROBIOLOGY, vol. 184, 29 February 2016 (2016-02-29), pages 84 - 93, XP029408402, ISSN: 0378-1135, DOI: 10.1016/j.vetmic.2016.01.010

## Description

### Technical Field

The invention relates to the field of medicines, and in particular to adefovir dipivoxil and adefovir for use in treating pseudorabies virus.

### Background

Pseudorabies virus (PRV), also known as porcine herpes virus type I, infectious bulbar palsy virus, scrapie virus, and Aujeszky's disease virus, is a member of the α-herpesvirus genus and its genome consists of about 150kb of DNA double strands, encoding at least 72 proteins. PRV infects a variety of mammals easily and causes them to death. Pigs are the natural hosts of PRV. PRV infects pigs and induces serious infectious diseases in the hosts, which brings huge economic losses to the pig breeding industry all over the world. Although the PRV vaccine has been widely used for a long time and the epidemic of the disease has been well controlled, in recent years, pseudorabies suddenly broke out in vaccinated pig farms in China and spread across the country, becoming one of the biggest hazards in the pig breeding industry of China.

At present, there is no specific therapeutic drug for PRV on the market, so it is of great significance to develop a specific and efficient therapeutic drug for PRV.

Adefovir dipivoxil has a chemical name of 9-[2-[bis(pivaloyloxy)methyl]phosphinyl]methoxy]ethyl]adenine; molecular formula: C₂₀H₃₂N₅O₈P; the structural formula is shown as follows:

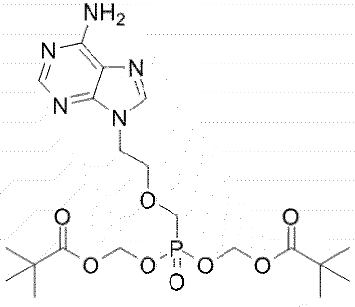

Adefovir dipivoxil is mainly indicated for the treatment of adult chronic hepatitis B patients with compensated liver function who have evidence of active hepatitis B virus replication and persistent elevation of serum amino acid transferase (ALT or AST) or histologically active lesions in the liver. After oral absorption, adefovir dipivoxil is rapidly converted into adefovir of an active structure (see the structural formula below) in the body to exert its medicinal effect.

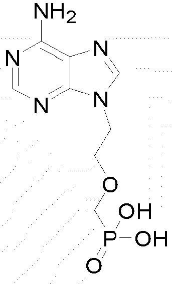

So far, the antiviral activity of adefovir dipivoxil or adefovir against PRV has not been reported.

Kuroda Yudai et al. (Kuroda, Y., et al., The Journal of Antibiotics 2019, 72 (12), 981-985, DOI: 10.1038/S41429-019-0234-4) relates to the antiviral effect of sinefungin on in vitro growth of feline herpesvirus type 1, showing that sinefungin specifically suppressed the replication of FHV-1 after its adsorption to the host feline kidney cells in a dose-dependent manner without obvious cytotoxicity to the host cells.

CN 109758487 A relates to the application of tuberous sword fern in controlling porcine pseudorabies and circoviridae mixed infection and goose paramyxovirus.

Angarano (Angarano, G. International Journal of Clinical Practice. Supplement 1999, 103, 39-41, ISSN: 1368-504X) relates to adefovir dipivoxil, having antiviral activity, and its use as first-line therapy.

CN 106974985 A relates to a method for preparing effective parts of tea saponin and new uses thereof against livestock virus, in particular to the application of the effective parts of tea saponin for preventing or treating the diseases of domestic animals infected by viruses, belonging to the field of medicinal chemistry.

CN 101781333 A relates to a crystalline form of adefovir dipivoxil and its preparation method.

WO 2007/068380 A1 relates to pharmaceutical compositions for treating virus infections and/or diseases caused thereby comprising 4{4-[3-(4-chloro-3-trifluoromethylphenyl)-ureido]-3-fluorophenoxy}-pyridine-2-carboxylic acid methylamide optionally combined with at least one additional therapeutic agent.

Zouharova Darina et al. (Zouharova, D., et al., Veterinary Microbiology 2016, 184, 84-93, DOI: 10.1016/j.vetmic.2016.01.010) relates to the anti-Pseudorabies virus activities of a series of synthetic diaminopurine-based acyclic nucleoside phosphonates.

### Summary of the Invention

The inventors unexpectedly found that adefovir dipivoxil and adefovir have strong antiviral activity against pseudorabies virus (PRV), thus providing the following invention.

According to the present invention this application provides a compound, a stereoisomer, solvate or pharmaceutically acceptable salt of the compound, or a solvate of the pharmaceutically acceptable salt of the compound, for use in treating pseudorabies virus infection or a disease related to pseudorabies virus infection;
the compound is adefovir or adefovir dipivoxil;
wherein the diseases related to pseudorabies virus infection is selected from pseudorabies disease such as pseudorabies disease that occurs in animals such as pigs, cattle, and sheep, and encephalitis, retinitis, or endophthalmitis related to pseudorabies virus infection.

Any reference in the description to a method of treatment of the human (or animal) body by therapy (or for diagnosis), to the use in a treatment, to a therapy is to be interpreted as reference to compounds, pharmaceutical compositions or combinations of the present invention for use in such a method.

According to the present invention the compound is adefovir or adefovir dipivoxil.

In some embodiments, the pharmaceutically acceptable salt of the compound is selected from hydrochloride, aspartate, taurate, gluconate, fructonate, salicylate, and maleate. In some embodiments, the pharmaceutically acceptable salt of the compound is adefovir dipivoxil hydrochloride.

In some embodiments, the solvate is a hydrate. In some embodiments, the solvate of the compound is an adefovir dipivoxil hydrate.

In some embodiments, the solvate of the pharmaceutically acceptable salt of the compound is a hydrate of a hydrochloride. In some embodiments, the solvate of the pharmaceutically acceptable salt of the compound is a hydrate of adefovir dipivoxil hydrochloride.

According to the present invention, this application provides a pharmaceutical composition for use in treating pseudorabies virus infection or a disease related to pseudorabies virus infection, wherein the pharmaceutical composition comprises the compound of the invention, or the stereoisomer, solvate or pharmaceutically acceptable salt of the compound, or a solvate of the pharmaceutically acceptable salt of the compound, and one or more pharmaceutically acceptable carriers or excipients; wherein the diseases related to pseudorabies virus infection is selected from pseudorabies disease such as pseudorabies disease that occurs in animals such as pigs, cattle, and sheep, and encephalitis, retinitis, or endophthalmitis related to pseudorabies virus infection; preferably, the pharmaceutical composition is made into a dosage form of tablet, capsule, pill, oral liquid preparation, granule, powder and injection;
preferably, the pharmaceutical composition is administered orally, injected, implanted, externally applied, sprayed or inhaled..

The pharmaceutically acceptable carriers comprise, but are not limited to: ion exchangers, alumina, aluminum stearate, lecithin, serum proteins such as human albumin, buffer substances such as phosphates, glycerol, sorbic acid, potassium sorbate, mixtures of part of glycerides of saturated vegetable fatty acids, water, salts or electrolytes such as protamine sulfate, disodium hydrogen phosphate, potassium hydrogen phosphate, sodium chloride, zinc salt, colloidal silica, magnesium trisilicate, polyvinylpyrrolidone, cellulosic substances, polyethylene glycol, sodium carboxymethylcellulose, polyacrylate, beeswax, and lanolin.

The excipients refer to additions other than a main drug in a pharmaceutical preparation. The excipients are stable in nature, have no incompatibility with the main drug, do not cause side effects, do not affect the curative effect, and are not easy to deform, crack, mildew, and be eaten by moth at room temperature. They are harmless and have no physiological effect to animals and human body, do not have chemical or physical reactions with the main drug, and do not affect the content determination of the main drug. The excipients can be binders, fillers, disintegrants, and lubricants in tablets; and preservatives, antioxidants, flavoring agents, fragrances, co-solvents, emulsifiers, solubilizers, osmotic pressure regulators, colorants, etc. in oral liquid preparations.

The pharmaceutical composition of the invention can be made into any pharmaceutically acceptable dosage form, such as tablet, capsule, pill, oral liquid preparation, granule, powder or injection or the like. The pharmaceutical composition of the invention can be administered orally, injected, implanted, externally applied, sprayed or inhaled.

The compound of the invention, the stereoisomer, solvate or pharmaceutically acceptable salt of the compound, or the solvate of the pharmaceutically acceptable salt of the compound can be used as an anti-infective therapeutic drug when used in the treatment of pseudorabies virus infection, optionally in combination with another therapeutic drug. Thus, in some embodiments, the pharmaceutical composition further comprises a second therapeutic drug.

In yet another aspect, this application provides the use of a compound of the invention, the stereoisomer, solvate or pharmaceutically acceptable salt of the compound, or a solvate of the pharmaceutically acceptable salt of the compound in combination with the second therapeutic drug in preparation of a medicament, wherein the medicament is used for treating pseudorabies virus infection or a disease related to pseudorabies virus infection.

In some embodiments, the second therapeutic drug used in the invention is selected from interferon drugs, broad-spectrum antiviral drugs, other therapeutic drugs for herpes virus, and anti-inflammatory drugs.

In some embodiments, the interferon drugs include, but are not limited to: α interferon, β interferon, γ interferon, etc.

In some embodiments, the broad-spectrum antiviral drugs include, but are not limited to: ribavirin, etc.

In some embodiments, other therapeutic drugs for herpes virus include, but are not limited to, acyclovir, ganciclovir, valacyclovir, etc.

In some embodiments, the anti-inflammatory drugs include, but are not limited to, ibuprofen, aspirin, indomethacin, etc.

In some embodiments of combined medication, the compound, the stereoisomer, solvate or pharmaceutically acceptable salt of the compound, or a solvate of the pharmaceutically acceptable salt of the compound and the second therapeutic drug can be in the same preparation unit or in different preparation units.

In some embodiments of combined medication, the compound, the stereoisomer, solvate or pharmaceutically acceptable salt of the compound, or a solvate of the pharmaceutically acceptable salt of the compound and the second therapeutic drug may be administered simultaneously or separately to a subject in need of treatment. The compound, the stereoisomer, solvate or pharmaceutically acceptable salt of the compound, or the solvate of the pharmaceutically acceptable salt of the compound may be administered first, and the second therapeutic drug is then administered after a certain period of time. Alternatively, the second therapeutic drug may be administered first, and the compound, the stereoisomer, solvate or pharmaceutically acceptable salt of the compound, or the solvate of the pharmaceutically acceptable salt of the compound is then administered after a certain period of time.

According to the present invention, this application provides a compound of the invention, the stereoisomer, solvate or pharmaceutically acceptable salt of the compound, or a solvate of the pharmaceutically acceptable salt of the compound, for use in treating pseudorabies virus infection or a disease related to pseudorabies virus infection.

In one aspect, this application provides a method for treating pseudorabies virus infection or a disease related to pseudorabies virus infection, including: administering to a subject in need a therapeutically effective dose of a compound of the invention, the stereoisomer, solvate or pharmaceutically acceptable salt of the compound, or a solvate of the pharmaceutically acceptable salt of the compound, or a pharmaceutical composition as described above.

In some embodiments, the subject is a mammal, including but not limited to: porcine, bovine, canine, mouse, rabbit, sheep, and human. In some embodiments, the subject is a domestic or wild animal (such as cattle, sheep, pig, dog, cat, and raccoon), or experimental animal (such as rabbit, guinea pig, and mouse), or human.

According to the present invention, the diseases related to pseudorabies virus infection is selected from pseudorabies disease such as pseudorabies disease that occurs in animals such as pigs, cattle, and sheep,
and encephalitis, retinitis, or endophthalmitis related to pseudorabies virus infection.

According to the present invention, this application provides a method for inhibiting pseudorabies virus (e.g., inhibiting the replication or proliferation of the pseudorabies virus), comprising: applying to the pseudorabies virus a compound of the invention, the stereoisomer, solvate or pharmaceutically acceptable salt of the compound, or a solvate of the pharmaceutically acceptable salt of the compound; the method is performed in vitro and for non-therapeutic purposes (e.g., scientific research).

In some embodiments, the method is performed in vitro and the compound, the stereoisomer, solvate or pharmaceutically acceptable salt of the compound, or a solvate of the pharmaceutically acceptable salt of the compound is used at a concentration of 0.05 to 5 µmol/L (e.g., 5 µmol/L, 2.5 µmol/L, 1.25 µmol/L, 0.625 µmol/L, 0.313 µmol/L, 0.156 µmol/L, or 0.078 µmol/L).

This application further provides use of a compound of the invention, or the stereoisomer, solvate or pharmaceutically acceptable salt of the compound, or a solvate of the pharmaceutically acceptable salt of the compound for preparing a preparation for inhibiting pseudorabies virus (e.g., inhibiting replication or proliferation of the pseudorabies virus).

This application further provides a compound of the invention, or the stereoisomer, solvate or pharmaceutically acceptable salt of the compound, or a solvate of the pharmaceutically acceptable salt of the compound, for use in inhibiting pseudorabies virus (e.g., inhibiting replication or proliferation of the pseudorabies virus).

### Definition of terms

In this application, unless otherwise specified, scientific and technical terms used herein have the meanings commonly understood by those skilled in the art. However, for a better understanding of the invention, definitions and explanations of some related terms are provided below. In addition, when the definitions and explanations of terms provided in this application are inconsistent with the meanings commonly understood by those skilled in the art, the definitions and explanations of terms provided in this application shall prevail.

In this application, "C₁-C₄ alkyl" refers to a linear or branched alkyl having 1 to 4 carbon atoms, including, for example, "C₁-C₃ alkyl ", "C₁-C₂ alkyl ", etc., such as methyl, ethyl, n-propyl, isopropyl, n-butyl, sec-butyl, tert-butyl, and isobutyl.

In this application, "halo" means substitution with one or more (e.g., 1, 2, 3 or 4) halogen atoms (e.g., fluorine, chlorine, bromine or iodine atoms).

In this application, "halo C₁-C₄ alkyl " refers to a group derived by substituting one or more hydrogen atoms on an C₁-C₄ alkyl with one or more halogen atoms, and the "halogen atom" and the "C₁-C₄ alkyl" are as defined above.

In this application, the "stereoisomerism" of a compound is divided into conformational isomerism and configurational isomerism, and the configurational isomerism is further divided into cis-trans isomerism and optical isomerism. Conformational isomerism refers to the stereoisomerism in which atoms or groups of the atoms in an organic molecule with a certain configuration have different arrangements in space due to the rotation or twisting of carbon-carbon single bonds. Common conformational isomerism comprises structures of alkanes and cycloalkanes, such as chair conformation and boat conformation of cyclohexane "Stereoisomer" means that the compound of the invention can be a racemate, a racemic mixture, a single enantiomer, a diastereomeric mixture, or a single diastereomer when the compound contains one or more asymmetric centers. The compound of the invention may have asymmetric centers, such asymmetric centers each independently give rise to two optical isomers, and the scope of the invention includes all possible optical isomers, diastereoisomeric mixtures, and pure or partially pure compounds. The compound of the invention may exist in a tautomeric form having different points of attachment of the hydrogen through displacement of one or more double bonds. For example, a ketone and its enol form are keto-enol tautomers. Each tautomer and mixtures thereof are included in the compounds of the invention. All enantiomers, diastereomers, racemates, cis-trans isomers, tautomers, geometric isomers, and epimers of the compound of the invention and their mixtures are included in the scope of the invention.

In this application, the "solvate" refers to a substance formed by association of a compound of the invention or a salt thereof with a solvent molecule. The solvent may be an organic solvent (such as methanol, ethanol, propanol, acetonitrile, etc.), water, and more. For example, the compound of the invention or a salt thereof may form a hydrate with water.

In this application, the "pharmaceutically acceptable salt" of a compound includes salts of acidic functional groups present in the compound with suitable inorganic or organic cations (bases), including salts of the acidic functional groups with alkali or alkaline earth metals, ammonium salts, and salts of the acidic functional groups with nitrogen-containing organic bases; also includes salts of basic functional groups (such as purine groups) present in the compound with suitable inorganic or organic anions (acids), including salts of the basic functional groups with inorganic or organic acids, such as salts of the basic functional groups with hydrochloric acid, hydrobromic acid, sulfuric acid, phosphoric acid, nitric acid, perchloric acid, fumaric acid, acetic acid, propionic acid, succinic acid, glycolic acid, formic acid, lactic acid, maleic acid, tartaric acid, citric acid, pamoic acid, malonic acid , hydroxymaleic acid, phenylacetic acid, glutamic acid, benzoic acid, salicylic acid, fumaric acid, toluenesulfonic acid, methanesulfonic acid, naphthalene-2-sulfonic acid, benzenesulfonic acid, hydroxynaphthoic acid, hydroiodic acid , aspartic acid, fructonic acid, gluconic acid, taurine, malic acid, tannic acid, or the like.

In this application, the "effective dose" refers to a dose sufficient to achieve, or at least partially achieve the desired effect. For example, a therapeutically effective amount refers to a dose sufficient to cure or at least partially arrest the disease and its complications in a patient already suffering from the disease. Determining such effective dose is well within the ability of those skilled in the art. For example, the dose effective for therapeutic use will depend on the severity of the disease to be treated, the general state of the patient's own immune system, the patient's general condition such as age, weight and sex, the mode of administration of the drug, other treatments taken concurrently, and more.

The dose of the compound of the invention, the stereoisomer, solvate or pharmaceutically acceptable salt, or a solvate of the pharmaceutically acceptable salt of the compound or the second therapeutic drug depends on the type and severity of the disease or condition and the characteristics of the subject, such as general health, age, sex, weight, and tolerance to the drug, and also on the type of the preparation and the mode of administration of the drug, as well as factors such as the period of administration or time interval. Those skilled in the art will be able to determine appropriate dosages based on these and other factors. In general, the daily dose of the compound of the invention, the stereoisomer, solvate or pharmaceutically acceptable salt of the compound, or a solvate of the pharmaceutically acceptable salt of the compound or the second therapeutic drug for treatment may be about 0.0001 to 1000 mg/kg (body weight)/day, and the daily dose may be administered at a time or in many times as needed.

### Beneficial Effect

Adefovir dipivoxil and adefovir have strong therapeutic activity against PRV infection, and can be used as therapeutic drugs for PRV. In addition, they have the advantages of easy acceptance by patients, low price and easy access. It is foreseeable that adefovir dipivoxil and adefovir will become a class of clinical drugs that can be taken for a long time and can effectively inhibit pseudorabies virus infection and the spread of pseudorabies virus and treat the pseudorabies virus infection.

### Brief Description of the Drawings

FIG. 1 shows the inhibitory effect of adefovir dipivoxil on pseudorabies virus replication in Example 1.
FIG. 2 shows the results of EC₅₀ assay on the inhibition of adefovir dipivoxil against pseudorabies virus in Example 1.
FIG. 3 shows that adefovir dipivoxil has no inhibitory activity against EB virus in Example 1.
FIG. 4 shows the inhibitory effect of adefovir dipivoxil on the release of pseudorabies virus from cells in Example 2.

### Detailed Description of Embodiments

The technical solutions in the embodiments of the invention will be described clearly and completely below in connection with the drawings in the embodiments of the invention, and it will be apparent that the embodiments described here are merely a part, not all of the embodiments of the invention. All other embodiments obtained by those of ordinary skill in the art based on the embodiments of the invention fall within the scope of the invention.

In the following embodiments, the used test materials and their sources are as follows:

Adefovir dipivoxil was purchased from MCE; high-glucose DMEM medium, fetal bovine serum FBS (Gibco), and 96-well plate (Nunc brand) were purchased from Thermo Company.

In the following Examples 1 to 2, adefovir dipivoxil was diluted with culture medium into a stock solution with a concentration of 10 mmol/L, and then diluted with culture medium to use concentrations of different gradients during use.

The cell line selected in the following examples is: porcine kidney cell PK-15 (culture medium is high-glucose DMEM medium+10% FBS, purchased from ATCC^{®} CCL-33).

### Example 1 Inhibitory effect of adefovir dipivoxil on pseudorabies virus replication

The experimental method was immunofluorescence method. The principle of this method is that according to the principle of antigen-antibody reaction, a known antigen or antibody is first labeled with a fluorescent group, and then the fluorescent antibody (or antigen) is used as a probe to check the corresponding antigen (or antibody) in cells or tissues. Fluorescence microscopy can be used to visualize the cells or tissues where the fluorescence is located, to determine the nature and localization of antigens or antibodies, and to determine the amount using quantitative techniques such as flow cytometry.

The specific experimental steps were as follows: the cells with a density of about 90% to 95% were digested with preheated trypsin and then re-suspended in a complete medium and pipetted sufficiently to obtain a single cell suspension and then counting was carried out. A 12-well plate for cell culture was prepared, a few drops of culture medium were first added in each well on the position where the slides were to be placed according to the size of the slides, and then the slides were placed on the droplets and pressed tightly so that the slides and the culture dish could be attached together by the tension of the medium to prevent the slides from floating when the cell suspension was added, and a double-layered cell patch would not be caused. The cells at an appropriate density were seeded into the 12-well plate. 24 hours or 48 hours later, according to the speed of cell growth, the cell density was observed and determined. After the12-well plate was full of cells, different concentrations of adefovir dipivoxil and 100 pfu of pseudorabies virus were then added in sequence, and immunofluorescence detection was then carried out 24 h after infection. During cell fixation, immunofluorescence and medium removal, PBS was generally added to wash the cells 3 times, 5 min each time. 1 ml of 4% paraformaldehyde was added to the wells for cell fixation, and the cells were fixed at room temperature for 20 min. The paraformaldehyde was removed by absorption, and rinsing with PBS was carried out three times, 5 min each time. 0.5% Triton X-100 (prepared in PBS) was added to the wells for permeabilization at room temperature for 20 min in order to permeabilize cells. The Triton X-100 was then removed and rinsing with PBS was carried out three times, 5 min each time. Blocking with 10% goat serum homologous to the secondary antibody (prepared in PBS) or with 5% BSA was carried out for 2 h (the selected blocking solution was the same as the antibody dilution used in the subsequent operation). Rinsing with PBS was not required after the blocking. The blocking solution was removed by absorption and a sufficient amount of the primary antibody with the appropriate concentration was added dropwise to each well (the antibody was used at a recommended concentration according to the operating instruction of the antibody at the first time, and the appropriate concentration of the antibody could be explored for subsequent stages of the experiment), and incubation was then carried out overnight in a humidified box at 4 °C. The primary antibody was removed by absorption and rinsing with PBS was carried out three times, 5 min each time. A sufficient amount of the secondary antibody with an appropriate concentration was added dropwise at 37 °C to the well, and incubation was then carried out for 1 h at room temperature in the dark. To be noted that the operation should be carried out in a dark place because the secondary antibody is labeled with fluorescein. The secondary antibody was removed by absorption and rinsing with PBS was carried out three times, 5 min each time. DAPI was added dropwise to the glass slide, or Hoechst was used to counterstain the nuclei, usually with blue fluorescence; incubation was then carried out in the dark for 5 to 10 min. Cells were gently rinsed three times with PBS, 5 min each time, to remove excess DAPI. To take the cell slide, because the cell slide was tightly connected with the bottom of the petri dish and the tension was large, a needle tip of a syringe can be bent to the back to make a small hook, so that the cell slide can be gently hooked up and taken out with small tweezers. The liquid on the cell slide was dried with absorbent paper, the cell slide was then blocked with a blocking solution containing an anti-fluorescence quencher (to be noted that the cell slide should be reversely attached to a poly-lysine slide). Then, observation and image acquisition were carried out under a fluorescence microscope. To be noted that an excitation light source corresponding to the antibody is selected.

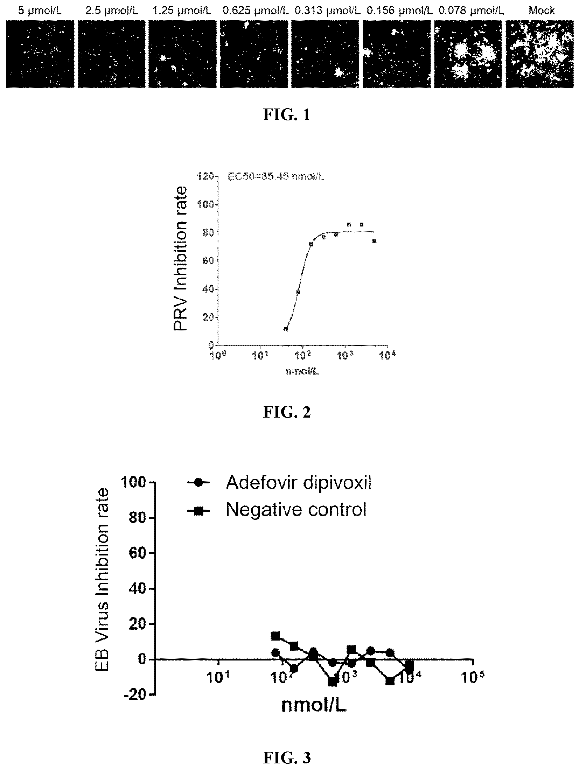

The inhibitory effect of adefovir dipivoxil on pseudorabies virus replication is shown in FIG. 1. The concentrations of adefovir dipivoxil used were 5 µmol/L, 2.5 µmol/L, 1.25 µmol/L, 0.625 µmol/L, 0.313 µmol/L, 0.156 µmol/L, and 0.078 µmol/L. As can be seen from FIG. 1, the adefovir dipivoxil of a high concentration can significantly inhibit the replication and proliferation of pseudorabies virus, confirming that adefovir dipivoxil has a significant proliferation inhibitory effect on pseudorabies virus.

The results of EC₅₀ assay on the inhibition of adefovir dipivoxil against pseudorabies virus are shown in FIG. 2. The concentrations of adefovir dipivoxil used were 5 µmol/L, 2.5 µmol/L, 1.25 µmol/L, 0.625 µmol/L, 0.313 µmol/L, 0.156 µmol/L, and 0.078 µmol/L. As can be seen from FIG. 2, the EC₅₀ of adefovir dipivoxil for the inhibition against pseudorabies virus proliferation reaches nanomolar level, confirming that adefovir dipivoxil has a good inhibitory effect on pseudorabies virus.

As a comparison, the inhibitory activity of adefovir dipivoxil against EB virus replication was tested, and the results are shown in FIG. 3. The concentrations of adefovir dipivoxil used were 5 µmol/L, 2.5 µmol/L, 1.25 µmol/L, 0.625 µmol/L, 0.313 µmol/L, 0.156 µmol/L, and 0.078 µmol/L. As can be seen from FIG. 3, adefovir dipivoxil has no inhibitory activity against EB virus which is also a herpes virus.

### Example 2 Inhibitory effect of adefovir dipivoxil on the release of pseudorabies virus from cells

The method used was virus plaque titration. According to the principle of this method, virus solution of each dilution is inoculated into a monolayer cell culture environment, after 2 hours of adsorption, the monolayer cells are covered with agarose, and the virus infects the cells and proliferates in the cells, causing the cells to rupture and die. Due to the limitations of the solid medium, the released virus can only spread around from the initially infected cells. After several proliferation cycles, a localized diseased cell area, i.e., the viral plaque, is formed. After staining with a dye, the living cells are red, while the cells in the plaque area are not stained, forming an unstained area.

The corresponding virus host cells were prepared and then digested evenly, and next the cell concentration was adjusted. The host cells were inoculated in a six-well plate at an appropriate concentration. After the cells grew into a monolayer, the culture medium was removed by absorption. The virus solution was prepared and diluted at a ten-fold gradient to obtain five solutions of virus with different concentrations. An appropriate amount of virus solution was added dropwise to each well and then absorbed for 2 h at 37 ° C, and extra virus solution was removed. 2% agarose solution with a low melting point was prepared and put in a water bath with a temperature of 40 °C to 50 °C for later use. The above agarose and 2× cell maintenance solution were mixed at a ratio of 1:1. The mixture was then added to the wells in a dose of 2 mL/well, and then cooled and solidified into a covering layer. The culture plate was placed upside down in a 37°C carbon dioxide incubator. 48 hours later, the cells were fixed with 10% formaldehyde for 30 min. The covering gel was then removed, and staining with 1% crystal violet was carried out for 15 min. After the dye solution was removed, the viral plaques were counted.

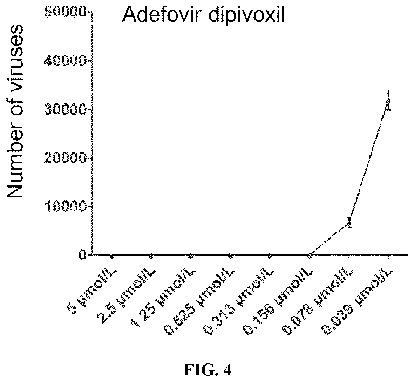

Different concentrations of adefovir dipivoxil and 100 pfu of pseudorabies virus were added to PK15 cells. After 24 hours of cultivation, the culture supernatant of PK15 cells was collected for virus plaque titration test. The results are shown in FIG. 4. The drug concentrations used were 5 µmol/L, 2.5 µmol/L, 1.25 µmol/L, 0.625 µmol/L, 0.313 µmol/L, 0.156 µmol/L, and 0.078 µmol/L. As can be seen from FIG. 4, adefovir dipivoxil can significantly inhibit the release of pseudorabies virus from cells.

The embodiments mentioned above are merely preferred embodiments of the invention and not intended to limit the invention.

## Claims

1. A compound, a stereoisomer, solvate or pharmaceutically acceptable salt of the compound, or a solvate of the pharmaceutically acceptable salt of the compound, for use in treating pseudorabies virus infection or a disease related to pseudorabies virus infection;
the compound is adefovir or adefovir dipivoxil;
wherein the diseases related to pseudorabies virus infection is selected from pseudorabies disease such as pseudorabies disease that occurs in animals such as pigs, cattle, and sheep,
and encephalitis, retinitis, or endophthalmitis related to pseudorabies virus infection.

2. The compound, the stereoisomer, solvate or pharmaceutically acceptable salt of the compound, or the solvate of the pharmaceutically acceptable salt of the compound for use according to claim 1, wherein the pharmaceutically acceptable salt is selected from hydrochloride, aspartate, taurate, gluconate, fructonate, salicylate, and maleate;
preferably, the pharmaceutically acceptable salt of the compound is adefovir dipivoxil hydrochloride.

3. The compound, the stereoisomer, solvate or pharmaceutically acceptable salt of the compound, or the solvate of the pharmaceutically acceptable salt of the compound for use according to claim 1 or 2, wherein the solvate is a hydrate;
preferably, the solvate of the compound is an adefovir dipivoxil hydrate;
preferably, the solvate of the pharmaceutically acceptable salt of the compound is a hydrate of a hydrochloride;
preferably, the solvate of the pharmaceutically acceptable salt of the compound is a hydrate of adefovir dipivoxil hydrochloride.

4. A pharmaceutical composition, for use in treating pseudorabies virus infection or a disease related to pseudorabies virus infection, wherein the pharmaceutical composition comprises the compound, the stereoisomer, solvate or pharmaceutically acceptable salt of the compound, or the solvate of the pharmaceutically acceptable salt of the compound according to any of claims 1 to 3, and one or more pharmaceutically acceptable carriers or excipients;
wherein the diseases related to pseudorabies virus infection is selected from pseudorabies disease such as pseudorabies disease that occurs in animals such as pigs, cattle, and sheep, and encephalitis, retinitis, or endophthalmitis related to pseudorabies virus infection;
preferably, the pharmaceutical composition is made into a dosage form of tablet, capsule, pill, oral liquid preparation, granule, powder and injection;
preferably, the pharmaceutical composition is administered orally, injected, implanted, externally applied, sprayed or inhaled.

5. The pharmaceutical composition for use according to claim 4, wherein the pharmaceutical composition further comprises a second therapeutic drug;
preferably, the second therapeutic drug is selected from: interferon drugs, such as α interferon, β interferon, and γ interferon; broad-spectrum antiviral drugs, such as ribavirin; other therapeutic drugs for herpes virus, such as acyclovir, ganciclovir, and valacyclovir; anti-inflammatory drugs such as ibuprofen, aspirin, and indomethacin;
preferably, the compound, the stereoisomer, solvate or pharmaceutically acceptable salt of the compound, or the solvate of the pharmaceutically acceptable salt of the compound and the second therapeutic drug are in the same preparation unit or in different preparation units.

6. The compound, the stereoisomer, solvate or pharmaceutically acceptable salt of the compound, or the solvate of the pharmaceutically acceptable salt of the compound for use according to any of claims 1 to 3 in combination with the second therapeutic drug, for use in treating pseudorabies virus infection or a disease related to pseudorabies virus infection;
wherein the diseases related to pseudorabies virus infection is selected from pseudorabies disease such as pseudorabies disease that occurs in animals such as pigs, cattle, and sheep, and encephalitis, retinitis, or endophthalmitis related to pseudorabies virus infection;
preferably, the second therapeutic drug is selected from: interferon drugs, such as α interferon, β interferon, and γ interferon; broad-spectrum antiviral drugs, such as ribavirin; other therapeutic drugs for herpes virus, such as acyclovir, ganciclovir, and valacyclovir; anti-inflammatory drugs such as ibuprofen, aspirin, and indomethacin;
preferably, the compound, the stereoisomer, solvate or pharmaceutically acceptable salt of the compound, or a solvate of the pharmaceutically acceptable salt of the compound and the second therapeutic drug are in the same preparation unit or in different preparation units.

7. A method for inhibiting pseudorabies virus, comprising: applying to the pseudorabies virus the compound, the stereoisomer, solvate or pharmaceutically acceptable salt of the compound, or the solvate of the pharmaceutically acceptable salt of the compound according to any of claims 1 to 3, wherein
the method is performed in vitro and for non-therapeutic purposes.

8. The compound, the stereoisomer, solvate or pharmaceutically acceptable salt of the compound, or the solvate of the pharmaceutically acceptable salt of the compound for use according to any of claims 1 to 3 for use in inhibiting pseudorabies virus on a mammal.

## Patentansprüche

1. Verbindung, Stereoisomer, Solvat oder pharmazeutisch unbedenkliches Salz der Verbindung oder ein Solvat des pharmazeutisch unbedenklichen Salzes der Verbindung zur Verwendung bei der Behandlung einer Pseudorabiesvirusinfektion oder einer mit einer Pseudorabiesvirusinfektion zusammenhängenden Erkrankung;
wobei die Verbindung Adefovir oder Adefovir-Dipivoxil ist;
wobei die mit einer Pseudorabiesvirusinfektion zusammenhängenden Erkrankungen aus der Pseudorabieserkrankung, wie etwa einer Pseudorabieserkrankung, die bei Tieren wie Schweinen, Rindern und Schafen auftritt,
und mit einer Pseudorabiesvirusinfektion zusammenhängenden Enzephalitis, Retinitis oder Endophthalmitis ausgewählt sind.

2. Verbindung, Stereoisomer, Solvat oder pharmazeutisch unbedenkliches Salz der Verbindung oder ein Solvat des pharmazeutisch unbedenklichen Salzes der Verbindung zur Verwendung nach Anspruch 1, wobei das pharmazeutisch unbedenkliche Salz aus Hydrochlorid, Aspartat, Taurat, Gluconat, Fructonat, Salicylat und Maleat ausgewählt ist;
vorzugsweise wobei es sich bei dem pharmazeutisch unbedenklichen Salz der Verbindung um Adefovir-Dipivoxil-Hydrochlorid handelt.

3. Verbindung, Stereoisomer, Solvat oder pharmazeutisch unbedenkliches Salz der Verbindung oder ein Solvat des pharmazeutisch unbedenklichen Salzes der Verbindung zur Verwendung nach Anspruch 1 oder 2, wobei das Solvat ein Hydrat ist;
vorzugsweise wobei es sich bei dem Solvat der Verbindung um Adefovir-Dipivoxil-Hydrat handelt;
vorzugsweise wobei es sich bei dem Solvat des pharmazeutisch unbedenklichen Salzes der Verbindung um ein Hydrat eines Hydrochlorids handelt;
vorzugsweise wobei es sich bei dem Solvat des pharmazeutisch unbedenklichen Salzes der Verbindung um Adefovir-Dipivoxil-Hydrochlorid handelt.

4. Pharmazeutische Zusammensetzung zur Verwendung bei der Behandlung einer Pseudorabiesvirusinfektion oder einer mit einer Pseudorabiesvirusinfektion zusammenhängenden Erkrankung, wobei die pharmazeutische Zusammensetzung die Verbindung, das Stereoisomer, Solvat oder ein pharmazeutisch unbedenkliches Salz der Verbindung oder das Solvat des pharmazeutisch unbedenklichen Salzes der Verbindung nach einem der Ansprüche 1 bis 3 sowie einen oder mehrere pharmazeutisch unbedenkliche Träger oder Hilfsstoffe umfasst;
wobei die mit einer Pseudorabiesvirusinfektion zusammenhängenden Erkrankungen aus der Pseudorabieserkrankung, wie etwa einer Pseudorabieserkrankung, die bei Tieren wie Schweinen, Rindern und Schafen auftritt, und mit einer Pseudorabiesvirusinfektion zusammenhängenden Enzephalitis, Retinitis oder Endophthalmitis ausgewählt sind;
vorzugsweise wobei die pharmazeutische Zusammensetzung zu einer Darreichungsform aus Tablette, Kapsel, Pille, oralen flüssigen Zubereitung, Granulat, Pulver oder Injektion verarbeitet wird;
vorzugsweise wobei die pharmazeutische Zusammensetzung oral verabreicht, injiziert, implantiert, äußerlich angewendet, gesprüht oder inhaliert wird.

5. Pharmazeutische Zusammensetzung zur Verwendung nach Anspruch 4, wobei die pharmazeutische Zusammensetzung ferner ein zweites Therapeutikum umfasst;
vorzugsweise wobei das zweite Therapeutikum ausgewählt ist aus: Interferonwirkstoffen, wie etwa α-Interferon, β-Interferon und γ-Interferon; Breitband-Antivirusmitteln wie etwa Ribavirin; anderen Therapeutika für Herpesviren wie etwa Aciclovir, Ganciclovir und Valaciclovir; entzündungshemmenden Arzneimitteln wie Ibuprofen, Aspirin und Indomethacin;
vorzugsweise wobei die Verbindung, das Stereoisomer, Solvat oder ein pharmazeutisch unbedenkliches Salz der Verbindung oder das Solvat des pharmazeutisch unbedenklichen Salzes der Verbindung und das zweite Therapeutikum sich in der gleichen Zubereitungseinheit oder in verschiedenen Zubereitungseinheiten befinden.

6. Verbindung, Stereoisomer, Solvat oder pharmazeutisch unbedenkliches Salz der Verbindung oder Solvat des pharmazeutisch unbedenklichen Salzes der Verbindung zur Verwendung nach einem der Ansprüche 1 bis 3 in Kombination mit dem zweiten Therapeutikum zur Verwendung bei der Behandlung einer Pseudorabiesvirusinfektion oder einer mit einer Pseudorabiesvirusinfektion zusammenhängenden Erkrankung;
wobei die mit einer Pseudorabiesvirusinfektion zusammenhängenden Erkrankungen aus der Pseudorabieserkrankung, wie etwa einer Pseudorabieserkrankung, die bei Tieren wie Schweinen, Rindern und Schafen auftritt, und mit einer Pseudorabiesvirusinfektion zusammenhängenden Enzephalitis, Retinitis oder Endophthalmitis ausgewählt sind;
vorzugsweise wobei das zweite Therapeutikum ausgewählt ist aus: Interferonwirkstoffen, wie etwa α-Interferon, β-Interferon und γ-Interferon; Breitband-Antivirusmitteln wie etwa Ribavirin; anderen Therapeutika für Herpesviren wie etwa Aciclovir, Ganciclovir und Valaciclovir; entzündungshemmenden Arzneimitteln wie Ibuprofen, Aspirin und Indomethacin;
vorzugsweise wobei die Verbindung, das Stereoisomer, Solvat oder ein pharmazeutisch unbedenkliches Salz der Verbindung oder ein Solvat des pharmazeutisch unbedenklichen Salzes der Verbindung und das zweite Therapeutikum sich in der gleichen Zubereitungseinheit oder in verschiedenen Zubereitungseinheiten befinden.

7. Verfahren zum Hemmen des Pseudorabiesvirus, umfassend: Anwenden der Verbindung, des Stereoisomers, Solvats oder pharmazeutisch unbedenklichen Salzes der Verbindung oder des Solvats des pharmazeutisch unbedenklichen Salzes der Verbindung nach einem der Ansprüche 1 bis 3, wobei das Verfahren in vitro und zu nicht therapeutischen Zwecken durchgeführt wird.

8. Verbindung, Stereoisomer, Solvat oder pharmazeutisch unbedenkliches Salz der Verbindung oder Solvat des pharmazeutisch unbedenklichen Salzes der Verbindung zur Verwendung nach Anspruch 1 bis 3, zur Verwendung beim Hemmen des Pseudorabiesvirus bei einem Säugetier.

## Revendications

1. Composé, stéréoisomère, solvate ou sel pharmaceutiquement acceptable du composé, ou solvate du sel pharmaceutiquement acceptable du composé, pour utilisation dans le traitement d'une infection par le virus de la pseudorage ou d'une maladie liée à une infection par le virus de la pseudorage ;
le composé est l'adéfovir ou l'adéfovir dipivoxil ;
dans lequel les maladies liées à l'infection par le virus de la pseudorage sont sélectionnées parmi une maladie d'Aujeszky, telle que la maladie d'Aujeszky qui survient chez les animaux tels que les porcs, le bétail et les moutons,
et l'encéphalite, la rétinite ou l'endophtalmie liées à une infection par le virus de la pseudorage.

2. Composé, stéréoisomère, solvate ou sel pharmaceutiquement acceptable du composé, ou solvate du sel pharmaceutiquement acceptable du composé pour utilisation selon la revendication 1, dans lequel le sel pharmaceutiquement acceptable est sélectionné parmi le chlorhydrate, l'aspartate, le taurate, le gluconate, le fructonate, le salicylate et le maléate ;
de préférence, le sel pharmaceutiquement acceptable du composé est le chlorhydrate d'adéfovir dipivoxil.

3. Composé, stéréoisomère, solvate ou sel pharmaceutiquement acceptable du composé, ou solvate du sel pharmaceutiquement acceptable du composé pour utilisation selon la revendication 1 ou 2, dans lequel le solvate est un hydrate ;
de préférence, le solvate du composé est un hydrate d'adéfovir dipivoxil ;
de préférence, le solvate du sel pharmaceutiquement acceptable du composé est un hydrate d'un chlorhydrate ;
de préférence, le solvate du sel pharmaceutiquement acceptable du composé est un hydrate de chlorhydrate d'adéfovir dipivoxil.

4. Composition pharmaceutique, pour utilisation dans le traitement d'une infection par le virus de la pseudorage ou d'une maladie liée à une infection par le virus de la pseudorage, dans laquelle la composition pharmaceutique comprend le composé, le stéréoisomère, le solvate ou le sel pharmaceutiquement acceptable du composé, ou le solvate du sel pharmaceutiquement acceptable du composé selon l'une quelconque des revendications 1 à 3, et un ou plusieurs supports ou excipients pharmaceutiquement acceptables ;
dans laquelle les maladies liées à l'infection par le virus de la pseudorage sont sélectionnées parmi la maladie d'Aujeszky, telle que la maladie d'Aujeszky qui survient chez des animaux tels que les porcs, le bétail et les moutons, et l'encéphalite, la rétinite ou l'endophtalmie liées à l'infection par le virus de la pseudorage ;
de préférence, la composition pharmaceutique est réalisée sous forme posologique de comprimé, gélule, pilule, préparation liquide orale, granulé, poudre et injection ;
de préférence, la composition pharmaceutique est administrée par voie orale, injectée, implantée, à usage externe, pulvérisée ou inhalée.

5. Composition pharmaceutique pour utilisation selon la revendication 4, dans laquelle la composition pharmaceutique comprend en outre un second médicament thérapeutique ;
de préférence, le second médicament thérapeutique est sélectionné parmi : les médicaments à base d'interférons, tels que l'interféron •, l'interf éron • et l'interféron • ; les médicaments antiviraux à large spectre, tels que la ribavirine ; d'autres médicaments thérapeutiques contre le virus de l'herpès, tels que l'acyclovir, le ganciclovir et le valacyclovir ; les médicaments anti-inflammatoires tels que l'ibuprofène, l'aspirine et l'indométhacine ;
de préférence, le composé, le stéréoisomère, le solvate ou le sel pharmaceutiquement acceptable du composé, ou le solvate du sel pharmaceutiquement acceptable du composé et le second médicament thérapeutique sont dans la même unité de préparation ou dans des unités de préparation différentes.

6. Composé, stéréoisomère, solvate ou sel pharmaceutiquement acceptable du composé, ou solvate du sel pharmaceutiquement acceptable du composé pour utilisation selon l'une quelconque des revendications 1 à 3 en combinaison avec le second médicament thérapeutique, pour utilisation dans le traitement d'une infection par le virus de la pseudorage ou d'une maladie liée à une infection par le virus de la pseudorage ;
dans lequel les maladies liées à l'infection par le virus de la pseudorage sont sélectionnées parmi la maladie d'Aujeszky, telle que la maladie d'Aujeszky qui survient chez des animaux tels que les porcs, le bétail et les moutons, et l'encéphalite, la rétinite ou l'endophtalmie liées à l'infection par le virus de la pseudorage ;
de préférence, le second médicament thérapeutique est sélectionné parmi : les médicaments à base d'interférons, tels que l'interféron •, l'interféron • et l'interféron • ; les médicaments antiviraux à large spectre, tels que la ribavirine ; d'autres médicaments thérapeutiques contre le virus de l'herpès, tels que l'acyclovir, le ganciclovir et le valacyclovir ; les médicaments anti-inflammatoires tels que l'ibuprofène, l'aspirine et l'indométhacine ;
de préférence, le composé, le stéréoisomère, le solvate ou le sel pharmaceutiquement acceptable du composé, ou un solvate du sel pharmaceutiquement acceptable du composé et le second médicament thérapeutique sont dans la même unité de préparation ou dans des unités de préparation différentes.

7. Procédé d'inhibition du virus de la pseudorage, comprenant : l'application du composé, du stéréoisomère, du solvate ou du sel pharmaceutiquement acceptable du composé, ou du solvate du sel pharmaceutiquement acceptable du composé selon l'une quelconque des revendications 1 à 3 sur le virus de la pseudorage, dans lequel
le procédé s'effectue in vitro et à des fins non thérapeutiques.

8. Composé, stéréoisomère, solvate ou sel pharmaceutiquement acceptable du composé, ou solvate du sel pharmaceutiquement acceptable du composé pour utilisation selon l'une quelconque des revendications 1 à 3 pour utilisation dans l'inhibition du virus de la pseudorage chez un mammifère.
